# EUROPEAN PATENT SPECIFICATION

(11) **EP 3 486 235 B1**
(45) Date of publication and mention of the grant of the patent: **18.11.2026**
(21) Application number: 16908488.6
(22) Date of filing: 15.07.2016
(51) Int. Cl.: C07D 249/12, C07C 251/74, C07D 213/76, C07D 401/04

(54) **SYNTHETIC METHOD FOR 1,2,4-TRIAZOLE-3-THIONE COMPOUND AND INTERMEDIATE THEREOF**
SYNTHETISCHES VERFAHREN FÜR DIE VERBINDUNG 1,2,4-TRIAZOL-3-THION UND DEREN ZWISCHENPRODUKT
PROCÉDÉ SYNTHÉTIQUE POUR LE COMPOSÉ 1,2,4-TRIAZOLE-3-THIONE ET SON INTERMÉDIAIRE

(43) Date of publication of application: 22.05.2019
(60) Divisional application: 26210473.0
(73) Proprietor: Oriental (Luzhou) Agrochemicals. Co., Ltd., Luzhou City, Sichuan 646300 (CN)
(72) Inventor: ZHANG, Zhiming, Luzhou city, Sichuan province 646300 (CN); DU, Xiaodong, Luzhou city, Sichuan province 646300 (CN); SHENG, Qiuju, Hangzhou, Zhejiang 310052 (CN); SHI, Jieping, Hangzhou, Zhejiang 310052 (CN); WANG, Junliang, Hangzhou, Zhejiang 310052 (CN); CHEN, Bangchi, Hangzhou, Zhejiang 310052 (CN)
(74) Representative: Haseltine Lake Kempner LLP
(86) International application number: PCT/CN2016/090129
(87) International publication number: WO 2018/010163

(56) References cited:
- CN-A- 1 274 346
- CN-A- 101 547 916
- CN-A- 105 636 441
- US-A- 4 659 644
- US-A1- 2007 111 984
- US-A1- 2014 364 414
- PENG ZHAN ET AL: "Arylazolylthioacetanilide. Part 8: Design, synthesis and biological evaluation of Novel 2-(2-(2,4-Dichlorophenyl)-2-1,2,4-triazol-3-ylthio)--arylacetamides As Potent HIV-1 inhibitors", EUROPEAN JOURNAL OF MEDICINAL CHEMISTRY, ELSEVIER, AMSTERDAM, NL, vol. 46, no. 10, 5 August 2011 (2011-08-05), pages 5039 - 5045, XP028390959, ISSN: 0223-5234, [retrieved on 20110812], DOI: 10.1016/J.EJMECH.2011.08.011
- MOSS G P ET AL: "Glossary of class names of organic compounds and reactive intermediates based on structure (IUPAC Recommendations 1995)", PURE & APPLIED CHEMISTRY, PERGAMON PRESS, OXFORD, GB, vol. 67, no. 8/9, 1 January 1995 (1995-01-01), pages 1307 - 1375, XP007914773, ISSN: 0033-4545, DOI: 10.1351/PAC199567081307
- VAILLANCOURT, V. A. ET AL.: "Synthesis and Biological Activity of Aminoguanidine and Diaminoguanidine Analogues of the Antidiabetic/Antiobesity Agent 3-Guanidinopropionic Acid", JOURNAL OF MEDICINAL CHEMISTRY, vol. 44, no. 8, 10 March 2001 (2001-03-10), pages 1231 - 1248, XP055456247, DOI: doi:10.1021/jm000094n
- GUAN, YUNFEI ET AL.: "A Review of Synthetic Method of Prothioconazole", AGROCHEMICALS, vol. 53, no. 9, 30 September 2014 (2014-09-30), pages 696 - 698, XP055570402, DOI: doi:10.16820/j.cnki.1006-0413.2014.09.023

## Description

### CROSS-REFERENCE TO RELATED APPLICATIONS

This application is a continuation of International Patent Application No. PCT/CN2016/090129, filed on July 15, 2016.

### TECHNICAL FIELD

The present disclosure relates to organic synthesis, and more specifically to a method of synthesizing 1,2,4-triazole-3-thione compounds and intermediates thereof.

### BACKGROUND

1,2,4-Triazole-3-thione compounds 1 is a class of materials with significant biological activities.

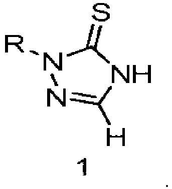

For example, it has been discovered that 1,2,4-triazole-3-thiones 1 have antibacterial activities (WO 2010142779, WO 2010146113, WO 2010146111, WO 2010146112, WO 2010040717, WO 2014167008, WO 2010040718, WO 9706152, W9638423, WO 9616048, WO 9641798, WO 9741107, WO 2010149758, EP 2746260, WO 2010146029, WO 2009077471, IN 193553, WO 2009077500 and WO 2014108288), antiviral activities (WO 2010149758, WO 2010122169, WO 2010142779, WO 2010149414 and WO 2010122167), antitumor activities (WO 2010149758, WO 2010122169, WO 2010142779, WO 2010149414 and WO 2010122167), and other biological activities (WO 2013047308, WO 2013084770 and WO 2011125317), and etc.

Strategy of synthesizing 1,2,4-triazole-3-thione compounds 1 can be classified into two categories according to the way that the triazole ring is constructed. The first strategy of producing target triazole-thione product is through cyclization of thiosemicarbazide with formic acid or formic acid derivatives. For example, reaction of N-amino-N-methylthiourea (compound 2, R is methyl) with formic acid gives 2-methyl-1,2,4-triazole-3-thione product (Justus Liebigs Annalen der Chemie, 643, 128-35; 1961). This method displays high efficiency in terms of the ring formation reaction, however, the preparation of compound 2 is very challenging, regioselectivity is usually a problem.

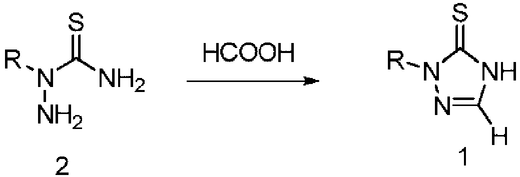

The second strategy of producing 1,2,4-triazole-3-thione compounds 1 is through modification of the already constructed triazole ring. For example, deprotonation of N-phenyltriazole (compound 3, R is phenyl) with lithium tert-butoxide followed by reaction with sulfur gives the target product (Journal of Organic Chemistry, 74(21), 8309-8313; 2009). This reaction requires the use of a strong organic base during the deprotonation processes, as well as manipulation under anhydrous and oxygen-free conditions, and regioselectivity of the deprotonation process is also a problem.

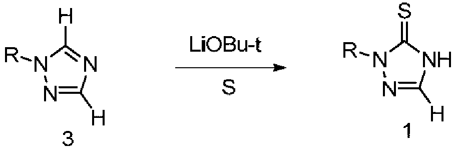

Heteroatom Chemistry, 14(1), 50-55; 2003 reported a method for the preparation of 1,2,4-triazole-3-thione compounds 1 using compound 4 through flash vacuum pyrolysis (FVP). This method requires not only high temperature for the reaction, but also results in a large amount of by-products at the same time.

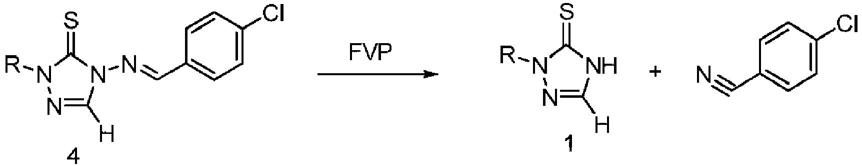

US 6559317 disclosed a deprotonation method for the preparation of 1,2,4-triazole-3-thione compound 1 using FeCl₃ to oxidize triazole 5. The starting material 5 of this method is unstable and difficult to prepare. In addition, treatment of the iron-containing waste is an environmental problem.

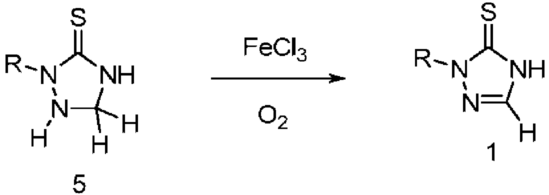

J Med Chem, 46(10), 5039-5045; 2011 describes the development of 2-(2-(2,4-dichlorophenyl-2*H*-1,2,4-triazol-3-ylthio)-*N*-arylacetamide derivatives.

US 2014/364414 A1 describes substituted imidazo[1,2-a]pyrazine derivatives which are inhibitors of the phosphodiesterase 10 enzyme (PDE10).

US 2007/111984 A1 describes bicyclic pyrimidinone compounds for the inhibition of HIV integrase.

US 4 659 644 A describes a diazo-type thermosensitive recording material which comprises a thermosensitive coloring layer formed on the support material, the thermosensitive coloring layer comprises a diazonium compound.

CN 105 636 441 A describes synthetic chemical processes for the preparation of pesticidal thioether and pesticidal sulfoxides.

CN 101 547 916 A describes the synthesis of pharmaceutical compositions comprising triazole, for mGluR5 receptor enhancers.

J Med Chem, 44(8), 1231-1248; 2001 describes the synthesis and biological activity of aminoguanidine and diaminoguanidine analogues of the antidiabetic/antiobesity agent 3-guanidinopropionic acid.

CN 1 274 346 A describes a process for the preparation of a triazolinthione derivative having microbicidal activity, in particular fungicidal activity.

Agrochemicals, 53(9), 696-698; 2014 describes a review of synthetic method of producing prothioconazole, a product obtained by multi-step reaction with 1-chlorocyclopropyl chloride and 1-acetyl-1-chlorocyclopropane.

### SUMMARY

The present application discloses a method of synthesizing 1,2,4-triazole-3-thione compounds and intermediates thereof in order to solve the problems in the prior art.

Method of synthesizing 1,2,4-triazole-3-thione compounds according to the present invention comprises the following steps:
step 1: reacting compound 6 or 6' with compound 7 or 7' to produce compound 8 or 8', as shown in the following reaction scheme: 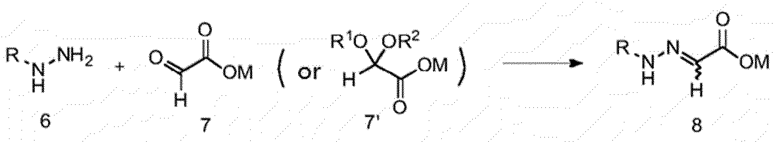 or 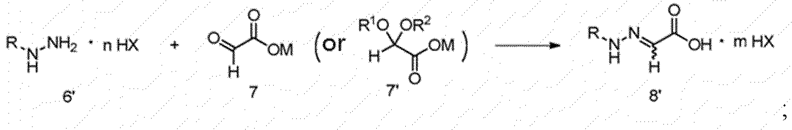 wherein:
   R is selected from a C₁-C₁₈ alkyl group, a C₆-C₁₈ aryl group or a C₅-C₆ heteroaryl group containing oxygen, nitrogen or sulfur; and R¹,R² is independently selected from hydrogen or a C₁-C₆ alkyl group, or R¹,R² taking together to form a ring is selected from dimethylene, trimethylene, tetramethylene or pentamethylene group;
   n is 0.5, 1 or 2;
   HX is selected from a hydrohalic acid, sulfuric acid or phosphoric acid;
   M is selected from hydrogen, an alkali metal or a NR³R⁴R⁵R⁶ group where R³, R⁴, R⁵ and R⁶ are independently hydrogen, a C₁-C₁₈ alkyl group or a C₆-C₁₈ aryl group; and
   m is 0 or 1;
step 2: reacting compound 8 or 8' with thiocyanate (M'SCN) to produce 1,2,4-triazole-3-thione compound 1, as shown in the following reaction scheme: 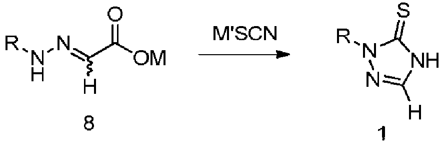 or 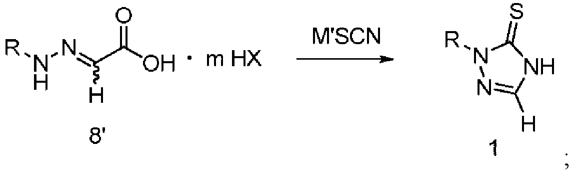 wherein:
   R is selected from a C₁-C₁₈ alkyl group, a C₆-C₁₈ aryl group or a C₅-C₆ heteroaryl group containing oxygen, nitrogen or sulfur;
   M is selected from hydrogen, an alkali metal or a NR³R⁴R⁵R⁶ group where R³, R⁴, R⁵ and R⁶ are independently hydrogen, a C₁-C₁₈ alkyl group or a C₆-C₁₈ aryl group;
   M' is an alkali metal or a NR³R⁴R⁵R⁶ group where R³, R⁴, R⁵ and R⁶ are independently hydrogen, a C₁-C₁₈ alkyl group or a C₆-C₁₈ aryl group;
   m is 0 or 1; and
   HX is selected from a hydrohalic acid, sulfuric acid or phosphoric acid.

Specifically, step 1 is carried out in the presence or absence of a solvent, and the solvent is selected from water, acetonitrile, tert-butanol, dichloromethane, DMF, DMSO and toluene or their mixture. A molar ratio of compound 6 or 6' to compound 7 or 7' is preferably 1:1-10. A reaction temperature in step 1 is preferably 0-40°C.

Step 2 is preferably carried out in the presence of an acid, and the acid can be used as a catalyst or directly as a solvent. The acid is preferably an organic acid selected from formic acid, acetic acid, trifluoroacetic acid, methanesulfonic acid and p-toluenesulfonic acid or their mixture. A molar ratio of the acid to compound 8 or 8' is preferably 0.01-100:1. The molar ratio of compound 8 or 8' to the thiocyanate in step 2 is preferably 1:1-5. A reaction temperature in step 2 is preferably 50-80°C.

Steps 1 and 2 can be carried out in a stepwise or a one-pot manner.

A method of synthesizing 1,2,4-triazole-3-thione compound 1, also according to the present invention, comprises the following step:
reacting compound 8 or 8' with thiocyanate (M'SCN) to produce 1,2,4-triazole-3-thione compound 1, as shown in the following reaction scheme: 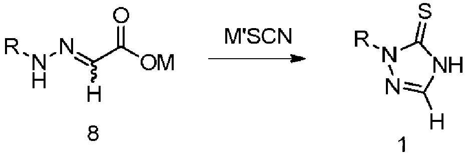 or 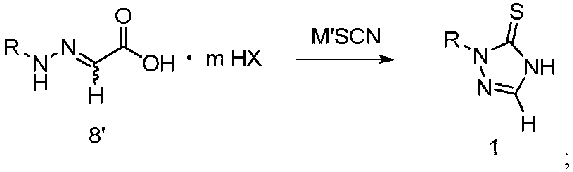
wherein:
   R is selected from a C₁-C₁₈ alkyl group, a C₆-C₁₈ aryl group or a C₅-C₆ heteroaryl group containing oxygen, nitrogen or sulfur;
   M is selected from hydrogen, an alkali metal or a NR³R⁴R⁵R⁶ group where R³, R⁴, R⁵ and R⁶ are independently hydrogen, a C₁-C₁₈ alkyl group or a C₆-C₁₈ aryl group;
   M' is selected from an alkali metal or a NR³R⁴R⁵R⁶ group where R³, R⁴, R⁵ and R⁶ are independently hydrogen, a C₁-C₁₈ alkyl group or a C₆-C₁₈ aryl group;
   m is 0 or 1; and
   HX is selected from a hydrohalic acid, sulfuric acid or phosphoric acid.

Specifically, this reaction is preferably carried out in the presence of an acid, and the acid can be used as a catalyst or directly as a solvent. The acid is preferably an organic acid selected from formic acid, acetic acid, trifluoroacetic acid, methanesulfonic acid and p-toluenesulfonic acid or their mixture. A molar ratio of the acid to compound 8 or 8' is preferably 0.01-100:1. The molar ratio of compound 8 or 8' to the thiocyanate is preferably 1: 1-5, and a reaction temperature is preferably 50-80°C.

The present application provides a compound 8: 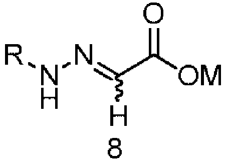 wherein:
R is selected from a C₁-C₁₈ alkyl group, a C₆-C₁₈ aryl group, or a C₅-C₆ heteroaryl group containing oxygen, nitrogen or sulfur, excluding a methyl group, a phenyl and a benzyl group; and
M is selected from an alkali metal or a NR³R⁴R⁵R⁶ group where R³, R⁴, R⁵ and R⁶ are independently hydrogen, a C₁-C₁₈ alkyl group or a C₆-C₁₈ aryl group;
or a compound which is selected from 2-{2-[2-(1-chlorocyclopropyl)-3-(2-chlorophenyl)-2-hydroxypropyl] hydrazono} acetic acid and 2-(3-chloropyridine hydrazono) acetic acid.

The present application further provides a compound 8': 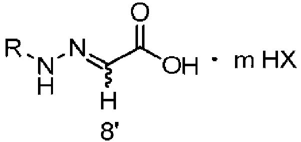 wherein:
R is selected from a C₁-C₁₈ alkyl group, a C₆-C₁₈ aryl group, or a C₃-C₆ heteroaryl group containing oxygen, or sulfur, excluding a methyl group, a phenyl and a benzyl group;
m is 1; and
HX is selected from a hydrohalic acid, sulfuric acid or phosphoric acid.

The present invention also provides a compound, which is 2-{2-[2-(1-chlorocyclopropyl)-3-(2-chlorophenyl)-2-hydroxypropyl] hydrazono} acetic acid hemisulfate.

A method of synthesizing 1,2,4-triazole-3-thione compounds and intermediates thereof has the following advantages:
1. The raw materials involved in the present invention are readily available, and the reaction is very specific in terms of regioselectivity, resulting in minimum byproduct formation and high product yield.
2. There are neither specific requirements for special equipment nor special operations such as high vacuum, high temperature, anhydrous and oxygen-free manipulations.
3. The process is simple and generates minimum wastes, suitable for industrial production.

### DETAILED DESCRIPTION OF EMBODIMENTS

The following embodiments are intended to illustrate the features of the present invention. The scope of the application is not limited to these embodiments.

### Example 1: synthesis of 2-(2-phenylhydrazono) acetic acid

To a 500 mL reaction flask were added 28.9 g of phenylhydrazine hydrochloride, 300 mL of water and 75 mL of acetonitrile. Then 29.6 g of 50% glyoxylic acid solution was added dropwise. The reaction mixture was stirred at room temperature. After the reaction was complete, the reaction mixture was filtered, washed with water and dried to give 29.0 g of product as a solid (88% yield).

¹H NMR (δ, DMSO-d6):11.125 (m, 1H), 7.283-7.252 (m, 2H), 7.157(s, 1H), 7.123-7.107 (d, 2H), 6.896-6.866 (t, 1H); MS: m/z =165.1 ([M+1]⁺).

### Example 2: synthesis of 2-(2-phenylhydrazono) acetic acid

To a 500 mL reaction flask were added 43.4 g of phenylhydrazine hydrochloride and 350 mL of water. Then 57.6 g of 50% sodium glyoxylate solution was added dropwise. The reaction mixture was stirred at room temperature. After the reaction was complete, the reaction mixture was filtered, washed with water and dried to give 45.3 g of product as a solid (92% yield).

### Example 3: synthesis of 2-(3-chloropyridine hydrazono) acetic acid

To a 100 mL reaction flask were added 5.0 g of 3-chloro-2-hydrazylpyridine and 50 mL of water. Then 26 g of 50% glyoxylic acid solution was added dropwise. The reaction mixture was stirred at room temperature. After the reaction was complete, the reaction mixture was filtered, washed with water and dried to give 6.6 g of product as a solid (95% yield).

¹H NMR (δ, DMSO-d6): 12.648 (s, 1H), 11.092 (s, 1H), 8.230-8.215 (m, 1H), 7.855-7.832 (m, 1H), 7.706 (s, 1H), 7.003-6.972 (m, 1H); MS: m/z =200.02 ([M+1]⁺).

### Example 4: synthesis of sodium 2-(2-phenylhydrazono) acetate

To a 100 mL reaction flask were added 5.0 g of phenylhydrazine and 50 mL of water. Then 8.9 g of 50% sodium glyoxylate solution was added dropwise. The reaction mixture was stirred at room temperature. After the reaction was complete, the solvent was removed. 7.6 g of a solid product was obtained by desiccation (100% yield).

### Example 5: synthesis of 2-(2-methylhydrazono) acetic acid

To a 100 mL reaction flask were added 5.0 g of methylhydrazine sulfate and 50 mL of water. Then 5.7 g of 50% glyoxylic acid solution was added dropwise. The reaction mixture was stirred at room temperature. After the reaction was complete, the reaction mixture was filtered, washed with water and dried to give 3.4 g of product as a solid (95% yield).

¹H NMR (δ, DMSO-d6): 11.975 (s, 1H), 6.463-6.461 (d, 1H), 4.995 (s, 1H), 2.835 (s, 3H); MS: m/z =103.0 ([M+1]⁺).

### Example 6: synthesis of 2-(2-benzylhydrazono) acetic acid

To a 250 mL reaction flask were added 11.7 g of benzylhydrazine dihydrochloride, 100 mL of water and 15 mL of acetonitrile. Then 9.5 g of 50% glyoxylic acid solution was added dropwise. The reaction mixture was stirred at room temperature. After the reaction was complete, the reaction mixture was filtered, washed with water and dried to give 9.4 g of product as a solid (88% yield).

¹H NMR (δ, DMSO-d6): 11.994 (s, 1H), 7.370-7.333 (t, 2H), 7.290-7.253 (t, 1H), 7.2256-7.208 (d, 2H), 6.397 (s, 1H), 5.339 (s, 1H), 4.700 (s, 2H); MS: m/z =179.1 ([M+1]⁺).

### Example 7: synthesis of 2-(2-benzylhydrazono) acetic acid

To a 50 mL reaction flask were added 2.34 g of benzylhydrazine dihydrochloride and 20 mL of water. Then 1.18 g of glyoxylic acid monohydrate was added in portions. The reaction mixture was stirred at room temperature. After the reaction was complete, the reaction mixture was filtered, washed with water and dried to give 1.94 g of product as a solid (91% yield).

### Example 8: synthesis of 2-{2-[2-(1-chlorocyclopropyl)-3-(2-chlorophenyl)-2-hydroxypropyl] hydrazono} acetic acid

To a 250 mL reaction flask were added 13.7 g of 2-(1-chlorocyclopropyl)-1-(2-chlorophenyl)-3-hydrazinopropan-2-ol and 100 mL of water. Then 4.7 g of glyoxylic acid monohydrate was added in portions. The reaction mixture was stirred at room temperature. After the reaction was complete, the reaction mixture was filtered, washed with water and dried to give 14.5 g of product as a solid (88% yield).

### Example 9: synthesis of sodium 2-(2-phenylhydrazono) acetate

To a 500 mL reaction flask were added 50 g of 2-(2-phenylhydrazono) acetic acid and 300 mL of ethanol. Then 24.4 g of a 50% sodium hydroxide solution was added dropwise. The reaction mixture was stirred at room temperature. After the reaction was complete, the reaction mixture was concentrated, slurried with ethanol, filtered and dried to give 56.1 g of product as a solid (99% yield).

### Example 10: synthesis of 2-{2-[2-(1-chlorocyclopropyl)-3-(2-chlorophenyl)-2-hydroxypropyl] hydrazono} acetic acid hemisulfate

To a 25 mL reaction flask were added 1.37 g of 2-(1-chlorocyclopropyl)-1-(2-chlorophenyl)-3-hydrazinopropan-2-ol and 15 mL of acetonitrile. Then 0.96 g of 50% glyoxylic acid solution and 0.25 g of concentrated sulfuric acid were added dropwise. The reaction mixture was stirred at room temperature. After the reaction was complete, the solvent was removed. 1.89 g of a solid product was obtained by lyophilization (99% yield).

### Example 11: synthesis of 2-{2-[2-(1-chlorocyclopropyl)-3-(2-chlorophenyl)-2-hydroxypropyl] hydrazono} acetic acid hemisulfate

To a 250 mL reaction flask were added 3.3 g of 2-{2-[2-(1-chlorocyclopropyl)-3-(2-chlorophenyl)-2-hydroxypropyl] hydrazono} acetic acid and 10 mL of acetonitrile. Then 0.5 g of concentrated sulfuric acid was added dropwise. The reaction mixture was stirred at room temperature. After the reaction was complete, the solvent was removed. 3.8 g of a solid product was obtained by lyophilization (99% yield).

### Example 12: synthesis of 2-{2-[2-(1-chlorocyclopropyl)-3-(2-chlorophenyl)-2-hydroxypropyl] hydrazono} acetic acid

To a 250 mL reaction flask was added 15.5 g of 2-(1-chlorocyclopropyl)-1-(2-chlorophenyl)-3-hydrazinopropan-2-ol hydrochloride. Then 75 g of 50% glyoxylic acid solution was added dropwise. The reaction mixture was stirred at room temperature. After the reaction was complete, the reaction mixture was filtered, washed with water and dried to give 16.4 g of product as a solid (99% yield).

¹H NMR (δ, CDCl₃): 7.456-7.437 (m, 1H), 7.405-7.386 (m, 1H), 7.271-7.252 (m, 1H), 7.245-7.223 (m, 1H), 6.971-6.899 (t, 1H), 6.781 (s, 1H), 3.906-3.898, 3.878-3.869 (dd, 1H), 3.547-3.519 (d, 1H), 3.519-3.508, 3.491-3.480 (dd, 1H), 3.211-3.183 (d, 1H), 2.506 (s, 2H), 1.175-1.134 (m, 1H), 0.964-0.852 (m, 3H); MS: m/z =330.9 ([M+1]⁺).

### Example 13: synthesis of 2-phenyl-1,2,4-triazole-3-thione

To a 250 mL reaction flask were added 4.9 g of 2-(2-phenylhydrazono) acetic acid, 8.7 g of potassium thiocyanate and 50 mL of trifluoroacetic acid. The reaction was heated to 50°C. After the reaction was complete, the reaction mixture was concentrated. 4.4 g of a solid product was obtained by adding with toluene, washing with water and distillation (83% yield).

¹H NMR (δ, DMSO-d6): 14.193 (s, 1H), 10.005 (s, 1H), 7.826-7.810 (d, 2H), 7.600-7.569 (m, 2H), 7.492-7.463 (m, 1H); MS: m/z =178.0 ([M+1]⁺).

### Example 14: synthesis of 2-benzyl-1,2,4-triazole-3-thione

To a 250 mL reaction flask were added 9 g of 2-(2-benzylhydrazono) acetic acid, 4 g of sodium thiocyanate and 100 mL of acetic acid. The reaction was heated to 80°C. After the reaction was complete, the reaction mixture was concentrated. 8.7 g of a solid product was obtained by adding with toluene, washing with water and distillation (91% yield).

¹HNMR (δ, DMSO-d6): 13.565 (s, 1H), 8.355 (s, 1H), 7.368-7.144 (m, 5H), 5.297 (s, 2H).

### Example 15: synthesis of 2-(3-chloropyridin-2-yl)-1,2,4-triazole-3-thione

To a 250 mL reaction flask were added 6.0 g of 2-(3-chloropyridinehydrazono) acetic acid hydrochloride, 11.4 g of ammonium thiocyanate and 50 mL of trifluoroacetic acid. The reaction was heated to 80°C. After the reaction was complete, the reaction mixture was concentrated. 5.0 g of a solid product was obtained by adding with toluene, washing with water and distillation (93% yield).

¹H NMR (δ, DMSO-d6): 7.904-7.889 (m, 1H), 7.568-7.545 (m, 1H), 6.564-6.533 (m, 1H), 6.234 (s, 2H); MS: m/z =213.0 ([M+1]⁺).

### Example 16: synthesis of 2-methyl-1,2,4-triazole-3-thione

To a 250 mL reaction flask were added 9.2 g of 2-(2-methylhydrazono) acetic acid, 14.6 g of sodium thiocyanate and 80 mL of acetic acid. The reaction was heated to 80°C. After the reaction was complete, the reaction mixture was concentrated. 9.3 g of a solid product was obtained by adding with toluene, washing with water and distillation (90% yield).

¹H NMR (δ, DMSO-d6): 8.733 (s, 1H), 6.039 (s, 1H), 3.631 (s, 3H).

### Example 17: synthesis of 2-phenyl-1,2,4-triazole-3-thione

To a 250 mL reaction flask were added 16.8 g of sodium 2-(2-phenylhydrazono) acetate, 14.6 g of sodium thiocyanate, 80 mL of acetic acid and 8.7 g of methanesulfonic acid. The reaction was heated to 80°C. After the reaction was complete, the reaction mixture was concentrated. 14.9 g of a solid product was obtained by adding with toluene, washing with water and distillation (93% yield).

### Example 18: synthesis of 2-[2-(1-chlorocyclopropyl)-3-(2-chlorophenyl)-2-hydroxypropyl]-1,2,4-triazole-3-thione

To a 250 mL reaction flask were added 16.5 g of 2-{2-[2-(1-chlorocyclopropyl)-3-(2-chlorophenyl)-2-hydroxypropyl] hydrazono} acetic acid, 6.1 g of sodium thiocyanate and 80 mL of acetic acid. The reaction was heated to 80°C. After the reaction was complete, the reaction mixture was concentrated. 16.3 g of a solid product was obtained by adding with toluene, washing with water and distillation (95% yield).

¹H NMR (δ, CDCl₃): 12.300 (s, 1H), 7.856 (s,1H), 7.549-7.544, 7.534-7.530 (dd, 1H), 7.377-7.374, 7.362-7.358 (dd, 1H), 7.242-7.183 (m, 2H), 4.802-4.773 (d, 1H), 4.510-4.481 (d, 1H), 4.212 (s, 1H), 3.621-3.594 (d, 1H), 3.193-3.166 (d, 1H), 0.943-0.922 (m, 1H), 0.885-0.767 (m, 3H); MS: m/z =343.9 ([M+1]⁺).

### Example 19: synthesis of 2-[2-(1-chlorocyclopropyl)-3-(2-chlorophenyl)-2-hydroxypropyl]-1,2,4-triazole-3-thione

To a 25 mL reaction flask were added 1.89 g of 2-{2-[2-(1-chlorocyclopropyl)-3-(2-chlorophenyl)-2-hydroxypropyl] hydrazono} acetic acid hemisulfate, 0.62 g of sodium thiocyanate and 10 mL of acetic acid. The reaction was heated to 50°C. After the reaction was complete, the reaction mixture was concentrated. 1.43 g of a solid product was obtained by adding with toluene, washing with water and distillation (83% yield).

### Example 20: synthesis of 2-[2-(1-chlorocyclopropyl)-3-(2-chlorophenyl)-2-hydroxypropyl]-1,2,4-triazole-3-thione

To a 100 mL reaction flask were added 0.93 g of 2-(1-chlorocyclopropyl)-1-(2-chlorophenyl)-3-hydrazinopropan-2-ol hydrochloride, 15 mL of acetonitrile, 0.55 g of a 50% glyoxylic acid solution and 0.24 g of sodium thiocyanate. The reaction was heated to 60°C. After the reaction was complete, 10 mL of water was added, the pH of the reaction mixture was adjusted to 2. The phases were separated, the aqueous phase was extracted using toluene, the organic phases were combined and concentrated to give 0.92 g of product as a solid (89% yield).

## Claims

1. A method of synthesizing 1,2,4-triazole-3-thione compound 1, comprising:
reacting compound 8 or 8' with thiocyanate (M'SCN) to produce 1,2,4-triazole-3-thione compound 1, as shown in the following reaction scheme: 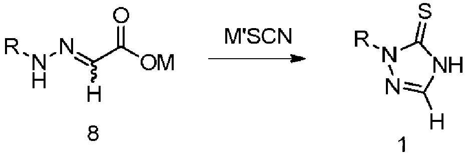 or 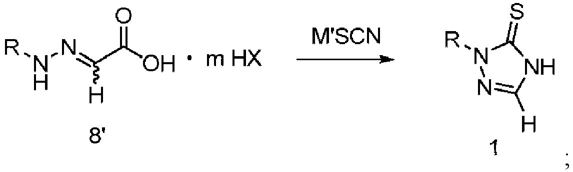
wherein:
R is selected from a C₁-C₁₈ alkyl group, a C₆-C₁₈ aryl group or a C₅-C₆ heteroaryl group containing oxygen, nitrogen or sulfur;
M is selected from hydrogen, an alkali metal or a NR³R⁴R⁵R⁶ group where R³, R⁴, R⁵ and R⁶ are independently hydrogen, a C₁-C₁₈ alkyl group or a C₆-C₁₈ aryl group;
M' is selected from an alkali metal or a NR³R⁴R⁵R⁶ group where R³, R⁴, R⁵ and R⁶ are independently hydrogen, a C₁-C₁₈ alkyl group or a C₆-C₁₈ aryl group;
m is 0 or 1; and
HX is selected from a hydrohalic acid, sulfuric acid or phosphoric acid.

2. The method of claim 1, wherein compound 8 or 8' is reacted with M'SCN in the presence of an acid; a molar ratio of compound 8 or 8' to M'SCN is 1: 1-5; and a reaction temperature is 50-80°C.

3. The method of claim 2, wherein the acid is an organic acid; and a molar ratio of the acid to compound 8 or 8' is 0.01-100:1.

4. The method of claim 3, wherein the organic acid is selected from formic acid, acetic acid, trifluoroacetic acid, methanesulfonic acid and p-toluenesulfonic acid or r mixture thereof.

5. The method of synthesizing 1,2,4-triazole-3-thione compounds 1 of any of claims 1 to 4 wherein compound 8 or 8' is produced by reacting compound 6 or 6' with compound 7 or 7' to produce compound 8 or 8', as shown in the following reaction scheme: 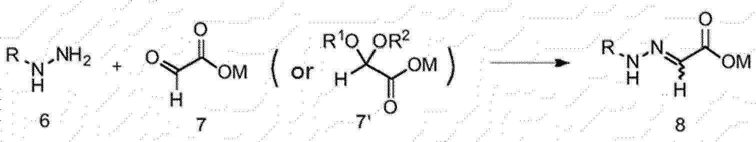 or 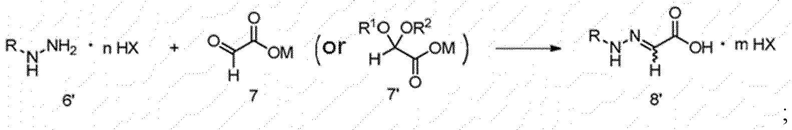 wherein:
R is selected from a C₁-C₁₈ alkyl group, a C₆-C₁₈ aryl group or a C₅-C₆ heteroaryl group containing oxygen, nitrogen or sulfur; and R¹,R² is independently selected from hydrogen or a C₁-C₆ alkyl group, or R¹,R² taking together to from a ring is selected from dimethylene, trimethylene, tetramethylene or pentamethylene;
n is 0.5, 1 or 2;
HX is selected from a hydrohalic acid, sulfuric acid or phosphoric acid;
M is selected from hydrogen, an alkali metal or a NR³R⁴R⁵R⁶ group where R³, R⁴, R⁵ and R⁶ are independently hydrogen, a C₁-C₁₈ alkyl group or a C₆-C₁₈ aryl group; and
m is 0 or 1.

6. The method of claim 5, wherein the reacting of compound 6 or 6' with compound 7 or 7' to produce compound 8 or 8' is carried out in the presence or absence of a solvent; a molar ratio of compound 6 or 6' to compound 7 or 7' is 1:1-10; and a reaction temperature in step 1 is 0-40°C.

7. The method of claim 6, wherein the solvent is selected from water, acetonitrile, tert-butanol, dichloromethane, DMF, DMSO and toluene or mixture thereof.

8. The method of claim 5, wherein the reacting of compound 6 or 6' with compound 7 or 7' to produce compound 8 or 8' (which may be termed 'step 1') and the reacting of compound 8 or 8' with thiocyanate (M'SCN) to produce 1,2,4-triazole-3-thione compound 1 (which may be termed 'step 2') is carried out in a stepwise or a one-pot manner.

9. A compound 8 shown as the following formula: 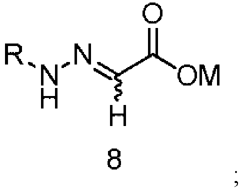 wherein:
R is selected from a C₁-C₁₈ alkyl group, a C₆-C₁₈ aryl group, or a C₅-C₆ heteroaryl group containing oxygen, nitrogen or sulfur, excluding a methyl group, a phenyl and a benzyl group; and
M is selected from an alkali metal or a NR³R⁴R⁵R⁶ group where R³, R⁴, R⁵ and R⁶ are independently hydrogen, a C₁-C₁₈ alkyl group or a C₆-C₁₈ aryl group.

10. A compound which is selected from 2-{2-[2-(1-chlorocyclopropyl)-3-(2-chlorophenyl)-2-hydroxypropyl] hydrazono} acetic acid and 2-(3-chloropyridine hydrazono) acetic acid.

11. A compound 8' shown as the following formula: 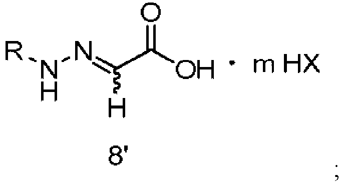 wherein:
R is selected from a C₁-C₁₈ alkyl group, a C₆-C₁₈ aryl group, or a C₅-C₆ heteroaryl group containing oxygen or sulfur, excluding a methyl group, a phenyl and a benzyl group;
m is 1; and
HX is selected from a hydrohalic acid, sulfuric acid or phosphoric acid.

12. A compound which is 2-{2-[2-(1-chlorocyclopropyl)-3-(2-chlorophenyl)-2-hydroxypropyl] hydrazono} acetic acid hemisulfate.

## Patentansprüche

1. Verfahren zur Synthese der Verbindung 1,2,4-Triazol-3-thion 1, umfassend:
Umsetzen einer Verbindung 8 oder 8' mit Thiocyanat (M'SCN), um die Verbindung 1,2,4-Triazol-3-thion 1 herzustellen, wie im folgenden Umsetzungsschema dargestellt: 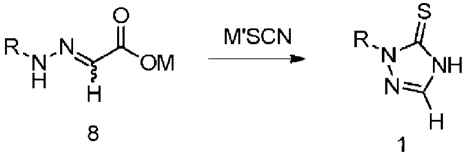 oder 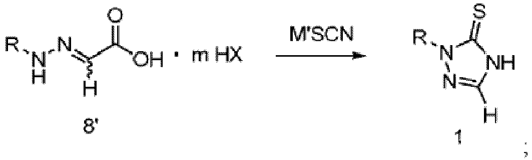
wobei:
R ausgewählt ist aus einer C₁-C₁₈-Alkylgruppe, einer C₆-C₁₈-Arylgruppe oder einer C₅-C₆-Heteroarylgruppe, die Sauerstoff, Stickstoff oder Schwefel enthält;
M ausgewählt ist aus Wasserstoff, einem Alkalimetall oder einer NR³R⁴R⁵R⁶-Gruppe, in der R³, R⁴, R⁵ und R⁶ unabhängig Wasserstoff, eine C₁-C₁₈-Alkylgruppe oder eine C₆-C₁₈-Arylgruppe sind;
M' ausgewählt ist aus einem Alkalimetall oder einer NR³R⁴R⁵R⁶-Gruppe, in der R³, R⁴, R⁵ und R⁶ unabhängig Wasserstoff, eine C₁-C₁₈-Alkylgruppe oder eine C₆-C₁₈-Arylgruppe sind;
m 0 oder 1 ist; und
HX ausgewählt ist aus einer Halogenwasserstoffsäure, Schwefelsäure oder Phosphorsäure.

2. Verfahren nach Anspruch 1, wobei die Verbindung 8 oder 8' in Gegenwart einer Säure mit M'SCN umgesetzt wird; ein Molverhältnis der Verbindung 8 oder **8'** zu M'SCN 1:1-5 beträgt; und eine Umsetzungstemperatur 50-80 °C beträgt.

3. Verfahren nach Anspruch 2, wobei die Säure eine organische Säure ist; und ein Molverhältnis der Säure zur Verbindung 8 oder 8' 0,01-100:1 beträgt.

4. Verfahren nach Anspruch 3, wobei die organische Säure ausgewählt ist aus Ameisensäure, Essigsäure, Trifluoressigsäure, Methansulfonsäure und p-Toluolsulfonsäure oder einer Mischung davon.

5. Verfahren zur Synthese von Verbindungen 1,2,4-Triazol-3-thion 1 nach einem der Ansprüche 1 bis 4, wobei die Verbindung 8 oder 8' durch Umsetzen einer Verbindung 6 oder 6' mit einer Verbindung 7 oder 7' hergestellt wird, um die Verbindung 8 oder 8' herzustellen, wie im folgenden Umsetzungsschema dargestellt: 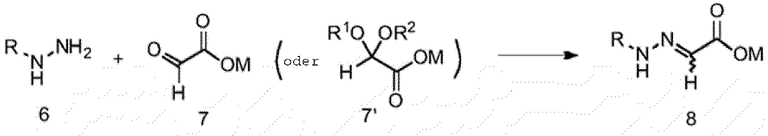 oder 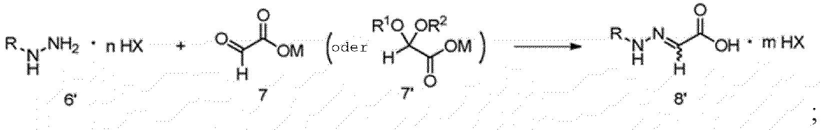 wobei:
R ausgewählt ist aus einer C₁-C₁₈-Alkylgruppe, einer C₆-C₁₈-Arylgruppe oder einer C₅-C₆-Heteroarylgruppe, die Sauerstoff, Stickstoff oder Schwefel enthält; und R¹,R² unabhängig ausgewählt ist aus Wasserstoff oder einer C₁-C₆-Alkylgruppe, oder R¹,R² zusammengenommen, um einen Ring auszubilden, ausgewählt ist aus Dimethylen, Trimethylen, Tetramethylen oder Pentamethylen;
n 0,5, 1 oder 2 ist;
HX ausgewählt ist aus einer Halogenwasserstoffsäure, Schwefelsäure oder Phosphorsäure;
M ausgewählt ist aus Wasserstoff, einem Alkalimetall oder einer NR³R⁴R⁵R⁶-Gruppe, in der R³, R⁴, R⁵ und R⁶ unabhängig Wasserstoff, eine C₁-C₁₈-Alkylgruppe oder eine C₆-C₁₈-Arylgruppe sind; und
m 0 oder 1 ist.

6. Verfahren nach Anspruch 5, wobei das Umsetzen der Verbindung 6 oder 6' mit der Verbindung 7 oder 7', um die Verbindung 8 oder 8' herzustellen, in Gegenwart oder Abwesenheit eines Lösungsmittels durchgeführt wird; ein Molverhältnis der Verbindung 6 oder 6' zur Verbindung 7 oder 7' 1:1-10 beträgt; und eine Umsetzungstemperatur in Schritt 1 0-40 °C beträgt.

7. Verfahren nach Anspruch 6, wobei das Lösungsmittel ausgewählt ist aus Wasser, Acetonitril, tert-Butanol, Dichlormethan, DMF, DMSO und Toluol oder einer Mischung davon.

8. Verfahren nach Anspruch 5, wobei das Umsetzen der Verbindung 6 oder 6' mit der Verbindung 7 oder 7', um die Verbindung 8 oder 8' herzustellen (das als ,Schritt 1' bezeichnet werden kann), und das Umsetzen der Verbindung 8 oder 8' mit Thiocyanat (M'SCN), um die Verbindung 1,2,4-Triazol-3-thion 1 herzustellen (das als ,Schritt 2' bezeichnet werden kann), schrittweise oder in einem Eintopfverfahren durchgeführt wird.

9. Verbindung 8, dargestellt durch die folgende Formel: 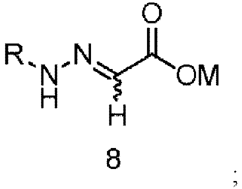 wobei:
R ausgewählt ist aus einer C₁-C₁₈-Alkylgruppe, einer C₆-C₁₈-Arylgruppe oder einer C₅-C₆-Heteroarylgruppe, die Sauerstoff,
Stickstoff oder Schwefel enthält, mit Ausnahme einer Methylgruppe, einer Phenyl- und einer Benzylgruppe; und
M ausgewählt ist aus einem Alkalimetall oder einer NR³R⁴R⁵R⁶-Gruppe, in der R³, R⁴, R⁵ und R⁶ unabhängig Wasserstoff, eine C₁-C₁₈-Alkylgruppe oder eine C₆-C₁₈-Arylgruppe sind.

10. Verbindung, die ausgewählt ist aus 2-{2-[2-(1-Chlorcyclopropyl)-3-(2-chlorphenyl)-2-hydroxypropyl]hydrazono}essigsäure und 2-(3-Chlorpyridinhydrazono)essigsäure.

11. Verbindung 8', dargestellt durch die folgende Formel: 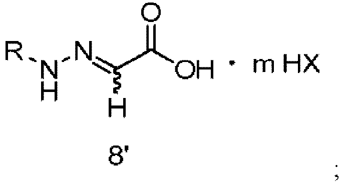 wobei:
R ausgewählt ist aus einer C₁-C₁₈-Alkylgruppe, einer C₆-C₁₈-Arylgruppe oder einer C₅-C₆-Heteroarylgruppe, die Sauerstoff oder Schwefel enthält, mit Ausnahme einer Methylgruppe, einer Phenyl- und einer Benzylgruppe;
m 1 ist; und
HX ausgewählt ist aus einer Halogenwasserstoffsäure, Schwefelsäure oder Phosphorsäure.

12. Verbindung, die 2-{2-[2-(1-Chlorcyclopropyl)-3-(2-chlorphenyl)-2-hydroxypropyl]hydrazono}essigsäurehemisulfat ist.

## Revendications

1. Procédé de synthèse du composé 1 de type 1,2,4-triazole-3-thione, comprenant :
le fait de faire réagir un composé 8 ou 8' avec du thiocyanate (M'SCN) pour produire le composé 1 de type 1,2,4-triazole-3-thione, comme indiqué dans le schéma réactionnel suivant : 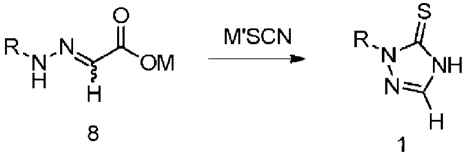 ou 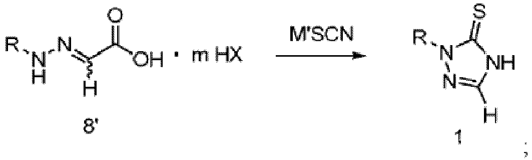
dans lequel :
R est choisi parmi un groupe alkyle en C₁-C₁₈, un groupe aryle en C₆-C₁₈ ou un groupe hétéroaryle en C₅-C₆ contenant de l'oxygène, de l'azote ou du soufre ;
M est choisi parmi l'hydrogène, un métal alcalin ou un groupe NR³R⁴R⁵R⁶ où R³, R⁴, R⁵ et R⁶ sont indépendamment de l'hydrogène, un groupe alkyle en C₁-C₁₈ ou un groupe aryle en C₆-C₁₈ ;
M' est choisi parmi un métal alcalin ou un groupe NR³R⁴R⁵R⁶ où R³, R⁴, R⁵ et R⁶ sont indépendamment de l'hydrogène, un groupe alkyle en C₁-C₁₈ ou un groupe aryle en C₆-C₁₈ ;
m vaut 0 ou 1 ; et
HX est choisi parmi un acide halohydrique, un acide sulfurique ou un acide phosphorique.

2. Procédé selon la revendication 1, dans lequel le composé 8 ou 8' réagit avec M'SCN en présence d'un acide ; un rapport molaire du composé 8 ou 8' à M'SCN est de 1:1-5 ; et une température de réaction est comprise entre 50 et 80 °C.

3. Procédé selon la revendication 2, dans lequel l'acide est un acide organique ; et un rapport molaire de l'acide au composé 8 ou 8' est compris entre 0,01 et 100:1.

4. Procédé selon la revendication 3, dans lequel l'acide organique est choisi parmi l'acide formique, l'acide acétique, l'acide trifluoroacétique, l'acide méthanesulfonique et l'acide p-toluènesulfonique ou un mélange r de ceux-ci.

5. Procédé de synthèse du composé 1 de type 1,2,4-triazole-3-thione selon l'une quelconque des revendications 1 à 4, dans lequel le composé 8 ou 8' est produit par réaction du composé 6 ou 6' avec le composé 7 ou 7' pour produire le composé 8 ou 8', comme indiqué dans le schéma réactionnel suivant : 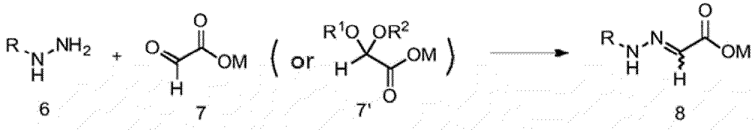 ou 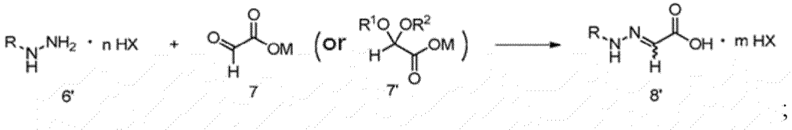 dans lequel :
R est choisi parmi un groupe alkyle en C₁-C₁₈, un groupe aryle en C₆-C₁₈ ou un groupe hétéroaryle en C₅-C₆ contenant de l'oxygène, de l'azote ou du soufre ; et R¹, R² est choisi indépendamment parmi l'hydrogène ou un groupe alkyle en C₁-C₆, ou R¹, R² forment ensemble un cycle choisi parmi le diméthylène, le triméthylène, le tétraméthylène ou le pentaméthylène ;
n vaut 0,5, 1 ou 2 ;
HX est choisi parmi un acide halohydrique, un acide sulfurique ou un acide phosphorique ;
M est choisi parmi l'hydrogène, un métal alcalin ou un groupe NR³R⁴R⁵R⁶ où R³, R⁴, R⁵ et R⁶ sont indépendamment de l'hydrogène, un groupe alkyle en C₁-C₁₈ ou un groupe aryle en C₆-C₁₈ ; et
m vaut 0 ou 1.

6. Procédé selon la revendication 5, dans lequel la réaction du composé 6 ou 6' avec le composé 7 ou 7' pour produire le composé 8 ou 8' est effectuée en présence ou en absence de solvant ; un rapport molaire du composé 6 ou 6' au composé 7 ou 7' est de 1:1-10 ; et une température de réaction à l'étape 1 est comprise entre 0 et 40 °C.

7. Procédé selon la revendication 6, dans lequel le solvant est choisi parmi l'eau, l'acétonitrile, le tert-butanol, le dichlorométhane, le DMF, le DMSO et le toluène ou un mélange de ceux-ci.

8. Procédé selon la revendication 5, dans lequel la réaction du composé 6 ou 6' avec le composé 7 ou 7' pour produire le composé 8 ou 8' (qui peut être appelée « étape 1 ») et la réaction du composé 8 ou 8' avec le thiocyanate (M'SCN) pour produire le composé 1 de type 1,2,4-triazole-3-thione (qui peut être appelée « étape 2 ») sont effectuées de manière progressive ou en une seule étape.

9. Composé 8 comme indiqué selon la formule suivante : 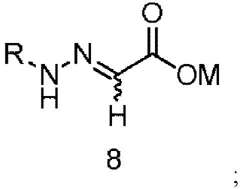 dans lequel :
R est choisi parmi un groupe alkyle en C₁-C₁₈, un groupe aryle en C₆-C₁₈ ou un groupe hétéroaryle en C₅-C₆ contenant de l'oxygène, de l'azote ou du soufre, à l'exclusion d'un groupe méthyle, d'un groupe phényle et d'un groupe benzyle ; et
M est choisi parmi un métal alcalin ou un groupe NR³R⁴R⁵R⁶ où R³, R⁴, R⁵ et R⁶ sont indépendamment de l'hydrogène, un groupe alkyle en C₁-C₁₈ ou un groupe aryle en C₆-C₁₈.

10. Composé sélectionné parmi l'acide 2-{2-[2-(1-chlorocyclopropyl)-3-(2-chlorophényl)-2-hydroxypropyl]hydrazono}acétique et l'acide 2-(3-chloropyridinehydrazono)acétique.

11. Composé 8' comme indiqué selon la formule suivante : 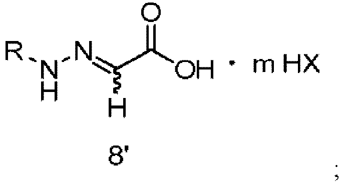 dans lequel :
R est choisi parmi un groupe alkyle en C₁-C₁₈, un groupe aryle en C₆-C₁₈ ou un groupe hétéroaryle en C_{S}-C₆ contenant de l'oxygène ou du soufre, à l'exclusion d'un groupe méthyle, d'un groupe phényle et d'un groupe benzyle ;
m vaut 1 ; et
HX est choisi parmi un acide halohydrique, un acide sulfurique ou un acide phosphorique.

12. Composé qui est l'hémisulfate d'acide 2-{2-[2-(1-chlorocyclopropyl)-3-(2-chlorophényl)-2-hydroxypropyl]hydrazono}acétique.
